# EUROPEAN PATENT APPLICATION

(11) **EP 4 318 486 A1**
(43) Date of publication of application: **07.02.2024**
(21) Application number: 23189220.9
(22) Date of filing: 02.08.2023
(51) Int. Cl.: G16H 20/30, A61B 5/00, A61B 5/11, G06V 40/00

(54) **MONITORING AND CORRECTION OF PHYSICAL EXERCISES INVOLVING PELVIC FLOOR MUSCLE CONTRACTION AND/OR LENGTHENING**

(30) Priority: 04.08.2022 EP 22398018
(71) Applicant: Sword Health, S.A., 4100-467 Porto (PT)
(72) Inventor: Ramos de Magalhães, Ivo Jorge, 4100-467 Porto (PT); Marques Gabriel, Ivo Emanuel, 4100-467 Porto (PT); Coelho Alves, José Carlos, 4100-467 Porto (PT); Ferreira Flores Martins, Cristina, 4100-467 Porto (PT); Helenurm, Mark Kaius, Utah, 84020 (US); Moutinho Colunas, Márcio Filipe, 4100-467 Porto (PT); Oliveira Santos, Pedro Henrique, 4100-467 Porto (PT); Dias Andrade, João Paulo, 4100-467 Porto (PT); Cardeano Pedrosa e Milheiro Maia, Marta Maria, 4100-467 Porto (PT); Ferro Bento, Virgílio António, 4100-467 Porto (PT)
(74) Representative: Balder IP Law, S.L.

(57) **Abstract**

Disclosed is a computer-implemented method. The method comprises processing, by at least one computing device, first measurements taken at least while a person performs a predetermined physical exercise, wherein the first measurements are taken by at least one motion tracking device, at least one optical sensor, or both. The method also comprises processing, by the at least one computing device, second measurements taken at least while the person performs the predetermined physical exercise, wherein the second measurements are taken by a pelvic sensor. The method also comprises determining, by at least one computing device, whether the person has performed the predetermined physical exercise based on the first measurements and the second measurements according to criteria associated with the predetermined physical exercise.

## Description

### CROSS-REFERENCE

This application claims priority to European Patent Application No. 22398018.6, filed August 4, 2022, which is entirely herein incorporated by reference.

### BACKGROUND

Pelvic floor disorder, PFD, is a medical condition that millions of people have. PFD affects muscles, tissues, ligaments and/or organs in the lower abdominal area of people.

Many illnesses or injuries that are part of PFD can be prevented from happening by adequate physical exercising, and recovered from by adequate physical exercising as well. In fact, recovery by way of physical rehabilitation speeds up the recovery process and improves the physical condition of the person. To this end, pelvic floor exercises include a range of exercises intended to lengthen and/or contract the pelvic floor muscle, along with the possible exercising of some body members or other muscles of the person by moving different parts of the body.

One of the problems of physical exercising is that by doing the physical exercises incorrectly, the person may get injured, the condition of the person may worsen, or, in the best-case scenario, the person does not exercise any part of the body thereby not improving her/his physical condition. There are a number of reasons why that might happen, such as: exercising of muscles different than those that may be exercised; excessive or insufficient tensioning of muscles, tendons and/or ligaments; etc.

Many people tend to do physical exercise incorrectly. Aside from the possibility of physically exercising with exercises not adequate to the condition of the person and, likewise, the possibility of forgetting what the exercises involve (i.e., which limbs have to be moved and how, and which others do not have to be moved), oftentimes the person does not have sufficient knowledge about the physical exercises and/or is unable to deduce whether the physical exercises that she/he has done have been correctly performed. These issues, in turn, prompt the people to conduct supervised physical exercising whereby a personal trainer or physical therapist instructs the person to do particular physical exercises, supervises the execution of the physical exercises, and corrects the execution if not properly executed.

Unsupervised physical exercising in the sense of not requiring a personal trainer or physical therapist for controlled physical exercising is beneficial as the person who is to physically exercise can do so in an autonomous, supervised manner. The appearance of systems capable of monitoring the person during physical exercising routines has enabled supervised physical exercising with no personal trainer or physical therapist, or at least without active supervision of the trainer or therapist, i.e. the trainer or therapist is assisted by the system so that she/he only needs to provide guidance to the user when there have been some issues in the physical exercising.

Existing systems may rely on motion trackers arranged on different body members of the user, and/or they rely on optical sensors, for example. These systems, however, cannot monitor the pelvic floor muscle in pelvic floor exercises and, hence, these exercises are still usually done in presence of a trainer or therapist to reduce the risk of getting injured. Even in the presence of trainers or therapists there is no certainty that problematic execution will be detected, or that the pelvic floor muscle will lengthen and/or contract in the prescribed manner.

### SUMMARY

In some aspects, described herein, are methods of monitoring and/or correcting physical exercises involving pelvic floor muscle movements. In some embodiments, the movements comprise contraction and/or lengthening of one or more pelvic floor muscles. In some embodiments, the methods are computer-implemented. In some embodiments, the methods comprise processing, by at least one computing device, first measurements taken at least while a person or subject performs a physical exercise.

In some embodiments, the physical exercise comprises both movement of one or more body members of the person or subject and at least one of lengthening and contracting of a pelvic floor muscle of the person or subject. In some embodiments, the first measurements are taken by at least one motion tracking device arranged on the person or subject to measure motion of at least the one or more body members, and/or at least one optical sensor arranged to capture images of at least a portion of the person or subject to measure motion of at least the one or more body members. In some embodiments, the methods comprise processing, by at least one computing device, second measurements taken at least while the person or subject performs the physical exercise. In some embodiments, the second measurements are taken by a pelvic sensor.

In some embodiments, the methods comprise determining, by at least one computing device, whether the person or subject has performed the physical exercise according to a set of criteria associated with the physical exercise by one or both: evaluating the processed first measurements to determine whether motion of the one or more body members fulfills at least one first criterion of the set of criteria; and evaluating the processed second measurements to determine whether the pelvic floor muscle lengthens and/or contracts such that it fulfills at least one second criterion of the set of criteria.

In some embodiments, the at least one first criterion is associated with motion of the one or more body members the at least one second criterion is associated with at least one of lengthening and contracting of a pelvic floor muscle. In some embodiments, the at least one computing device determines that the person or subject has correctly performed the physical exercise when all criteria of the set of criteria have been fulfilled. In some embodiments, the methods comprise processing, by at least one computing device, first measurements taken at least while a person or subject performs a physical exercise. In some embodiments, the first measurements are taken by at least one motion tracking device, at least one optical sensor, or both.

In some embodiments, the methods comprise processing, by the at least one computing device, second measurements taken at least while the person or subject performs the physical exercise, wherein the second measurements are taken by a pelvic sensor. In some embodiments, the methods comprise determining, by at least one computing device, whether the person or subject has performed the physical exercise based on the first measurements and the second measurements according to criteria associated with the physical exercise. In some embodiments, the physical exercise comprises both movement of one or more body members of the person or subject and at least one of lengthening or contracting of a pelvic floor muscle of the person or subject. In some embodiments, the at least one motion tracking device is configured to be arranged on the person or subject to measure motion of at least the one or more body members. In some embodiments, the at least one optical sensor is configured to capture images or video of the person or subject to measure motion of at least the one or more body members.

In some embodiments, the criteria comprises at least one first criterion associated with motion of the one or more body members and at least one second criterion associated with at least one of lengthening or contracting of the pelvic floor muscle. In some embodiments, determining whether the person or subject has performed the exercise comprises evaluating the processed first measurements to determine whether the motion of the one or more body members fulfills the at least one first criterion of the criteria.

In some embodiments, determining whether the person or subject has performed the exercise comprises evaluating the processed second measurements to determine whether the pelvic floor muscle at least lengthens or contracts such that it fulfills the at least one second criterion of the criteria. In some embodiments, evaluating the processed second measurements to determine whether the pelvic floor muscle at least lengthens or contracts such that it fulfills the at least one second criterion of the criteria comprises: calculating at least one ratio of the pelvic floor muscle lengthening or contracting thereby calculating how much the person or subject lengthened or contracted the pelvic floor muscle.

In some embodiments, determining whether the person or subject has performed the physical exercise further comprises: evaluating the processed first and second measurements to determine whether the motion of the one or more body members and the at least one of lengthening or contracting of the pelvic floor muscle fulfill at least one third criterion of the criteria. In some embodiments, the at least one third criterion at least requires that the at least one of lengthening or contracting of the pelvic floor muscle must occur during performance of the physical exercise. In some embodiments, the at least one first criterion comprises one or more of: at least one body member exceeding a orientation or being within a orientation range, at least one body member exceeding an acceleration or being within an acceleration range, at least one body member exceeding an angular velocity or being within an angular velocity range, and at least one body member being still or substantially still with an orientation for a period of time.

In some embodiments, the methods comprise providing, by the at least one computing device, one or more instructions or signals indicative of incorrect performance of the physical exercise when at least one criterion of the of criteria has not been fulfilled according to the determining step. In some embodiments, the methods comprise transmitting, by the at least one computing device to another computing device, data representative of incorrect performance of the physical exercise when at least one criterion of the set of criteria has not been fulfilled.

In some embodiments, the methods comprise modifying or adjusting, by the at least one computing device, the criteria by disabling one or more criteria thereof, or by changing values of one or more criteria thereof. In some embodiments, the one or more instructions or signals are indicative of at least one of: which criterion have not been fulfilled; and guidance on how to move the one or more body members or the pelvic floor muscle to fulfill the criterion that have not been fulfilled. In some embodiments, at least one of the first measurements or further measurements are taken by the at least one optical sensor, and the method further comprises processing, by the at least one computing device, the at least one of the first measurements or further measurements taken at least while the person or subject performs the physical exercise.

In some embodiments, determining whether the person or subject has performed the physical exercise further comprises evaluating the processed measurements of the at least one optical sensor to determine whether motion of one or more joints of the person or subject fulfills at least one further criterion of the criteria. In some embodiments, the first measurements are at least taken by the at least one motion tracking device. In some embodiments, one or each motion tracking device of the at least one motion tracking device comprises an accelerometer and a gyroscope.

In some embodiments, the pelvic sensor further measures motion of a pelvic floor of the person or subject, wherein the determination of whether the person or subject has performed the physical exercise further comprises evaluating the processed second measurements to determine whether motion of the pelvic floor fulfills at least one further criterion of the criteria. In some embodiments, the physical exercise further comprises lack of motion of at least one body member of the person or subject. In some embodiments, the first measurements further measure motion of the at least one body member. In some embodiments, determining whether the person or subject has performed the physical exercise further comprises evaluating the processed first measurements to determine whether the motion of the at least one body member fulfills at least one criterion of the criteria associated with lack of motion.

In some embodiments, the methods comprise processing, by the at least one computing device, a third measurement taken by the pelvic sensor while the person or subject keeps the pelvic floor muscle relaxed, a fourth measurement taken by the pelvic sensor while the person or subject lengthens the pelvic floor muscle, or a fifth measurement by the pelvic sensor while the person or subject contracts the pelvic floor muscle. In some embodiments, the methods comprise calibrating, by the at least one computing device, the processed second measurements or at least a portion of the criteria based on the third measurement and at least one of the fourth measurement or the fifth measurement.

In some embodiments, the methods comprise retrieving, by the at least one computing device, data associated with the person or subject, wherein the data are representative of at least one of a relaxed state, a lengthened state, or a contracted state of the pelvic floor muscle of the person or subject. In some embodiments, calibrating, by the at least one computing device, the processed second measurements or the at least one second criterion based on the data representative of at least one of the relaxed state, the lengthened state, or the contracted state of the pelvic floor muscle of the person or subject.

In another aspect, described herein are motion tracking systems for monitoring and/or correcting physical exercises involving pelvic floor muscle movements. In some embodiments, the systems comprise at least one motion tracking device or at least one optical sensor. In some embodiments, the systems comprise a pelvic sensor. In some embodiments, the systems comprise a computing device comprising a processor. In some embodiments, the processor is operative to: process first measurements taken at least while a person or subject performs a physical exercise, wherein the first measurements are taken by at least one motion tracking device, at least one optical sensor, or both; process second measurements taken at least while the person or subject performs the physical exercise, wherein the second measurements are taken by a pelvic sensor; and/or determine whether the person or subject has performed the physical exercise based on the first measurements and the second measurements according to criteria associated with the physical exercise. In some embodiments, the systems described herein are configured to perform any of the methods described herein.

A first aspect of the disclosure relates to a method comprising: processing first measurements taken at least while a person performs a predetermined physical exercise, where: the predetermined physical exercise comprises both movement of one or more body members of the person and at least one of lengthening and contracting of a pelvic floor muscle of the person; and the first measurements are taken by at least one motion tracking device arranged on the person to measure motion of at least the one or more body members, and/or at least one optical sensor arranged to capture images of at least a portion of the person to measure motion of at least the one or more body members; processing second measurements taken at least while the person performs the predetermined physical exercise, where the second measurements are taken by a pelvic sensor; and determining whether the person has performed the predetermined physical exercise according to a predetermined set of criteria associated with the predetermined physical exercise by both: evaluating the processed first measurements to determine whether motion of the one or more body members fulfills at least one first criterion of the predetermined set of criteria, where the at least one first criterion is associated with motion of the one or more body members; and evaluating the processed second measurements to determine whether the pelvic floor muscle lengthens and/or contracts such that it fulfills at least one second criterion of the predetermined set of criteria, where the at least one second criterion is associated with at least one of lengthening and contracting of a pelvic floor muscle; and the determination that the person has correctly performed the predetermined physical exercise is when all criteria of the predetermined set of criteria have been fulfilled.

At least one computing device conducts the processing and determination for automatic supervised physical exercising by the person without the presence of a trainer or therapist.

The at least one computing device receives the first and second measurements from a motion tracking system that the computing device(s) may or may not be part of. The motion tracking system provides the measurements from at least two different types of sensors: a pelvic sensor, and at least one of optical sensor(s) and motion tracker(s).

By processing the measurements of the pelvic sensor, the computing device(s) establishes how much the pelvic floor muscle contracts/lengthens, and when that happens. Likewise, by processing the measurements of the at least other sensor (which can be a set of sensors when at least the motion tracker or trackers are part of the motion tracking system, with each motion tracker being arrangeable on a different part of the body of the person for monitoring several body members), the computing device(s) establishes the movements performed by the person.

The combined evaluation of the processed first and second measurements enables the determination, by the computing device(s), of whether the person has performed the predetermined physical exercise according to the predetermined set of criteria.

The computing device(s) determines that performance of the predetermined physical exercise is correct when the predetermined set of criteria is fulfilled since exercises involving the lengthening/contracting of the pelvic floor muscle (i.e. pelvic floor exercises) require both the correct physical exercising of the pelvic floor muscle and correct physical exercising of body members or other muscles. When at least one criterion of the predetermined set of criteria is not fulfilled, irrespective of whether it is one of the first criteria and/or one of the second criteria, the computing device(s) determines that performance of the predetermined physical exercise is incorrect.

As soon as the computing device(s) processes the measurements, the computing device(s) can determine the correct/incorrect performance of the physical exercise. This, on the one hand, means that when the computing device(s) receives the measurements, it may start the processing and determination, so prompt reception of the measurements from the sensors, for example by way of a wireless and/or wired connection, permits prompt determination, even in real time or almost in real time. And, on the other hand, it also means that the determination is possible even when the person has not completed the physical exercise yet, i.e., the predetermined movement or predetermined set of movements encompassed in one or more repetitions of the physical exercise; it is noted that the predetermined physical exercise may encompass one or more repetitions of a same predetermined movement to be performed by the person. The computing device(s) thus may progressively check whether the exercise is correctly performed as it processes fresh measurements when they are received in different packets or continuously, and/or check the correct performance of the exercise upon processing all the first and second measurements, for example after some time has elapsed since the beginning of the performance of the physical exercise.

In some embodiments, the method further comprises: processing third, fourth and fifth measurements respectively taken while the person keeps the pelvic floor muscle relaxed, while the person lengthens the pelvic floor muscle, and while the person contracts the pelvic floor muscle, thereby providing data associated with the person representative of: the pelvic floor muscle of the person being in relaxed state; the pelvic floor muscle of the person being in lengthened state; and the pelvic floor muscle of the person being in contracted state; with the third, fourth and fifth measurements being taken by the pelvic sensor; and calibrating the processed second measurements or the at least one second criterion, the calibration being based on: the data representative of the pelvic floor muscle being in the relaxed state; and at least one of: the data representative of the pelvic floor muscle being in the lengthened state; and the data representative of the pelvic floor muscle being in the contracted state.

For a more accurate determination of whether the person correctly exercises the pelvic floor muscle according to the physical exercise(s) to be done, the computing device(s) carries out a calibration process in which the pelvic sensor provides measurements of the pelvic floor muscle in several states.

The pelvic floor muscle in relaxed state is a state that the muscle will typically be at some point during the physical exercise, which can be at the beginning of the exercise, and/or at the end, and/or in between the beginning and the end of the exercise. The lengthened and contracted states will be indicative of possible lengthening and contracting of the pelvic floor muscle and will preferably be indicative of maximum lengthening and contracting of the pelvic floor muscle that the person is able to achieve. Hence, the person preferably achieves as much lengthening and contracting during the calibration process so that the amount of lengthening and/or contracting of the pelvic floor muscle when the person is doing the predetermined physical exercise can be evaluated by the computing device(s) in relation to the maximum lengthening/contracting.

When the person tries to prevent PFD or recover from it, the physical exercise(s) may not require maximum lengthening and/or contracting of the pelvic floor muscle but rather just a fraction of it (e.g., a percentage of the maximum value achievable by the person), and sometimes this fraction of lengthening and/or contracting is adjusted in the predetermined physical exercises in accordance with the physical condition of the person. What is more, the maximum lengthening and/or contracting tend/s to vary as the physical condition of the person changes, thus the calibration process makes possible a more adequate evaluation of the exercising of the pelvic floor muscle.

For the measurements of the pelvic floor muscle in the different states, the person is preferably in a set of predetermined calibration positions or postures, for example but without limitation, a set in which the person lies on a surface, the person standing upright, the person sitting on a surface, etc. In each set, the person has a predetermined calibration position or posture for the pelvic floor muscle to be in relaxed state, a predetermined calibration position or posture for the pelvic floor muscle to be in lengthened state, and a predetermined calibration position or posture for the pelvic floor muscle to be in contracted state; the positions or postures for the three states could be the same, or two or all be different.

To this end, for an adequate calibration process, in some embodiments, the method further comprises commanding provision of one or more user perceptible signals at least representative of the set of predetermined calibration positions or postures and/or guidance for the person to be according to each calibration position or posture of the set. Additionally or alternatively, in some embodiments, the method further comprises processing further measurements taken at least while the person is according to each calibration position or posture of the set (i.e. while the person keeps the pelvic floor muscle relaxed, while the person lengthens the pelvic floor muscle, and while the person contracts the pelvic floor muscle), with the further measurements being taken by the at least one motion tracking device and/or the at least one optical sensor; and determining whether the person has been according to each calibration position or posture of the set by evaluating the processed further measurements. The evaluation may be conducted such that, for example, one or more predetermined criteria are set for each calibration position or posture, and the processed further measurements shall be indicative of each criterion of each one or more predetermined criteria being fulfilled.

As part of the processing of the third, fourth and fifth measurements, the computing device(s) may apply techniques thereto such as averaging or filtering to at least partially remove data that shows imperfectness in the execution of the calibration positions or postures by the person. People for instance make subtle or even coarse movements while holding a particular calibration position or posture due to spasms, fatigue, etc. Such movements potentially introduce noise in the calibration process. The pelvic sensor could also introduce noise in the measurements owing to imperfect electronics or disturbances introduced by the environment.

In some embodiments, the method further comprises registering data associated with the person, the data being representative of the registered data are representative of the pelvic floor muscle of the person being in relaxed state; the pelvic floor muscle of the person being in lengthened state; and the pelvic floor muscle of the person being in contracted state.

As part of the calibration process, the calibration values obtained are stored, for example in a memory of the computing device(s), a server remote from the computing device(s), etc., for posterior retrieval and calibration of the measurements associated with the pelvic floor muscle.

In some embodiments, the method further comprises: retrieving registered data associated with the person, the registered data being representative of: the pelvic floor muscle of the person being in relaxed state; the pelvic floor muscle of the person being in lengthened state; and the pelvic floor muscle of the person being in contracted state; calibrating the processed second measurements or the at least one second criterion, the calibration being based on: the data representative of the pelvic floor muscle being in the relaxed state; and at least one of: the data representative of the pelvic floor muscle being in the lengthened state; and the data representative of the pelvic floor muscle being in the contracted state.

On many occasions, the same equipment is shared among a plurality of users, so when a calibration process wants to be conducted for a given user, previous values can be retrieved for calibration, thereby not requiring the user to conduct the entire calibration process all over again.

In some embodiments, the evaluation of the processed second measurements to determine whether the lengthening and/or contracting of the pelvic floor muscle fulfills the at least one second criterion comprises calculating at least one ratio of the pelvic floor muscle lengthening and/or contracting thereby calculating how much the person lengthened and/or contracted the pelvic floor muscle based on the calibrated relaxed and the lengthened and/or contracted states thereof, where the at least one ratio is calculated based on: the data representative of the pelvic floor muscle being in the relaxed state; and the data representative of the pelvic floor muscle being in the lengthened state and/or the contracted state; and the at least one second criterion comprises at least one lengthening and/or contracting ratio of the pelvic floor muscle.

With the calibrated measurements of the pelvic floor muscle, the computing device(s) can calculate the ratio/s for establishing different levels of pelvic floor muscle contracting and/or lengthening.

In some embodiments, the determination of whether the person has performed the predetermined physical exercise further comprises: evaluating the processed first and second measurements to determine whether the motion of the one or more body members and the lengthening and/or contracting of the pelvic floor muscle fulfill at least one third criterion of the predetermined set of criteria, where the at least one third criterion is at least associated with a sequence in which motion of the one or more body members and the at least one of lengthening and contracting of a pelvic floor muscle must occur during performance of the predetermined physical exercise.

In addition to the monitoring of the motion of the one or more body members themselves, and the lengthening/contracting of the pelvic floor muscle, many physical exercises require some coordination between the two, meaning that it is not enough that the motion and the lengthening/contracting exist.

The first and second measurements include, at least in preferred embodiments, data indicative of the time when the measurement was taken and/or when a packet with the respective packet was created. Any of the two can be used for evaluation of the sequence followed by the person while doing the exercise.

The third criterion or criteria define how the two parts of the physical exercise must be reproduced by the person in terms of temporal sequence.

The evaluation of the actual motion of the body member(s) and lengthening/contracting of the pelvic floor muscle is performed by means of the first and second criteria. Notwithstanding, in some embodiments, the third criterion or criteria may be further associated with the motion and/or the at least one of lengthening and contracting in the sense that the criterion/criteria establish/es how much motion and/or lengthening/contracting must occur at particular time instants or situations while the person does the predetermined exercise. By way of example, at half of the complete rotation of one of the legs of the person, the lengthening of the pelvic floor muscle shall be more than a predetermined threshold, or within a predetermined range, for a corresponding third criterion to the met.

In some embodiments, the method further comprises commanding the pelvic sensor and/or the at least one motion tracking device to vibrate whenever, during the determination of whether the person has performed the predetermined physical exercise, it is determined that at least one first criterion (related to motion of one or more body members) has been fulfilled and both at least one second criterion (related to lengthening/contracting of the pelvic floor muscle) and at least one third criterion (related to the time sequence between motion of body members and lengthening/contracting of the pelvic floor muscle) have yet to be fulfilled.

The pelvic sensor and/or the motion tracking device(s) may include a vibrating device. For assisting the person to lengthen and/or contract the pelvic floor muscle at that moment in time, or at those moments in time, when the exercise requires the extension and/or contraction of the muscle, at least one data packet may be transmitted from the computing device(s) to the respective device with the command to actuate the vibrating device. The vibration notifies the person that the pelvic floor muscle must be exercised.

In some embodiments, the method further comprises commanding provision of one or more user perceptible signals at least representative of incorrect performance of the predetermined physical exercise when at least one criterion of the predetermined set of criteria has not been fulfilled according to the determining step. Additionally or alternatively, the method further comprises transmitting, to a computing device, data representative of incorrect performance of the predetermined physical exercise when at least one criterion of the predetermined set of criteria has not been fulfilled. Additionally or alternatively, the method further comprises modifying the predetermined set of criteria by disabling one or more criteria thereof, and/or by changing values of one or more criteria thereof.

Feedback indicating that the physical exercise is not properly executed might be provided to the person for warning the person about the incorrect execution and, thus, possible risk of getting injured, so that the person may stop exercising (or at least stop performing that predetermined physical exercise and exercise according to a different one) or be more careful when trying to reproduce the motion of body members and the lengthening/contracting of the pelvic floor muscle more in subsequent repetitions.

The computing device(s) may likewise or alternatively transmit a notification to a different computing device to inform a predetermined person, for example a personal trainer or a therapist monitoring the activity of the person or the evolution of the person over time, or personnel in charge of maintenance of the motion tracking system. For example, the trainer or a physical therapist can be notified of the incorrect execution so that she/he may contact the person to find out e.g. whether the person is in a condition that requires immediate medical attention, different exercises need to be prescribed, etc.; or the personnel may have the duty of revising and possibly repairing the motion tracking system in case the performance of the exercises is determined as incorrect due to a malfunctioning sensor.

It is also possible for the computing device(s) to, instead of the above actions or in addition to any one of the above actions, change the predetermined set of criteria to make them less demanding for the person that is exercising. By way of example, a criterion that for instance requires the person to stay with a posture for a period of time may be modified to reduce the amount of time, or be disabled altogether. In that case, the person will continue physically exercising but in a more relaxed manner as less efforts will be demanded from the person. Consequently, the computing device(s) will be determining correct performance of the predetermined physical exercise more often as it will be more likely that, because of the exercising by the person, all the criteria will be met. This possible relaxation of the criteria is convenient especially whenever the person has not met one or more criteria during repetitions of the exercise several times, even more so when it is several times in a row, which is a sign of too demanding criteria or the person being too tired. By relaxing the criteria, the person can follow with the exercising or physical rehabilitation in a manner that is tailored to the condition of the person.

In some embodiments, the one or more user perceptible signals comprise indications of: which predetermined criterion or criteria have not been fulfilled; and/or guidance on how to move the one or more body members and/or the pelvic floor muscle to fulfill the predetermined criterion or criteria that have not been fulfilled.

The setting of multiple criteria for the same physical exercise not only permits the evaluation of several different parameters relating to the body members and the pelvic floor muscle, but also the determination of what the person does wrong during the execution of the physical exercise. It can happen that most of the exercise is correctly executed but the person is e.g., leaning forward when not having to, moving the hip to the side when not having to, not moving the hip to the side when having to, etc., and as part of the automatic digital supervision, guidance to the person can be provided for correcting the execution of the physical exercise. That way, the person receives the necessary input to progressively improve the execution and reduce the mistakes therein.

In some embodiments, either: the first measurements are at least taken by the at least one optical sensor; or the method further comprises processing further measurements taken at least while the person performs the predetermined physical exercise, where the further measurements are taken by the at least one optical sensor; the determination of whether the person has performed the predetermined physical exercise further comprises evaluating the processed measurements of the at least one optical sensor to determine whether motion of one or more joints of the person fulfills at least one further criterion of the predetermined set of criteria, where the at least one further criterion is associated with motion of the one or more joints.

Optical sensor(s) takes measurements, namely images or video footage, of the person for processing by the computing device(s). The processing of these measurements is for monitoring how joints of the person move during the performance of the predetermined physical exercise, which can also be relevant for the correct performance.

The measurements may be processed with digital processing techniques known in the art configured to identify joints of a person when images or video footage capture the shape of a person. For instance, but without limitation, digital libraries and algorithms like visual feature analysis, Google's TensorFlow Pose estimation, Google's MediaPipe Pose and BlazePose, the algorithm described in "Reconstructing 3D Human Pose from 2D Image Landmarks" by V. Ramakrishna, etc., are several known techniques for joint identification and tracking.

With one or more joints have been identified and tracked, the at least one computing device evaluates the motion thereof to determine whether the physical exercise is correctly executed. To this end, one or more further (e.g., third, fourth, etc.) criteria associated with the physical exercise and the person may be fulfilled by the motion of the joint(s). By way of example, one criterion may be that the hip joint is at a height equal to or lower than the knee joint of one of the legs when the person raises that leg.

In some embodiments in which calibration is conducted, the method further comprises processing sixth, seventh and eighth measurements respectively taken while the person keeps the pelvic floor muscle relaxed, while the person lengthens the pelvic floor muscle, and while the person contracts the pelvic floor muscle, thereby providing data associated with the person representative of: the pelvic floor muscle of the person being in relaxed state; the pelvic floor muscle of the person being in lengthened state; and the pelvic floor muscle of the person being in contracted state. In these embodiments, the calibration is conducted when it is determined that the person has been in a set of predetermined calibration positions or postures corresponding to each of keeping the pelvic floor muscle relaxed, lengthening the pelvic floor muscle, and contracting the pelvic floor muscle.

With the measurements, the computing device(s) makes sure that the person has been in predetermined positions or postures during the calibration procedure for accurate calibration of the measurements or criteria related to the pelvic floor muscle. The sixth, seventh and eighth measurements may be taken by the at least one optical sensor and/or by the at least one motion tracking device. Any one of both types of measurements enable the computing device(s) to check the positions or postures of the person and compare it with the predetermined ones.

In some embodiments, the first measurements are at least taken by the at least one motion tracking device; each motion tracking device of the at least one motion tracking device at least comprising an accelerometer and a gyroscope. In some embodiments, each motion tracking device further comprises a magnetometer.

Acceleration and orientation (and, possibly, magnetic field) measurements of motion tracking device(s) arranged on body members of the person can be processed by the at least one computing device to digitally determine and provide a motion tracking sequence of at least the body members where the at least one motion tracking device is arranged. The computing device(s) has identification data of the motion tracking device(s) with which it establishes where on the body of the person each motion tracker is arranged, and the measurements provide information about how the concerned body member is oriented and how it moves. By combining the data of all motion tracking devices or using the data of the motion tracking device when there is just one, the computing device(s) provides an overall motion tracking sequence of at least part of the body of the person.

In some embodiments, the pelvic sensor further measures motion of a pelvic floor of the person; where the determination of whether the person has performed the predetermined physical exercise further comprises evaluating the processed second measurements to determine whether motion of the pelvic floor fulfills at least one further criterion of the predetermined set of criteria, where the at least one further criterion is associated with motion of the pelvic floor.

In addition to measurements for determining the lengthening and/or contracting of the pelvic floor muscle, the pelvic sensor measures motion of the pelvic floor with inertial measurement sensors of the pelvic sensor.

The motion of the pelvic sensor may also be important in the execution of the predetermined physical exercise, in which case one further (e.g., third, fourth, etc.) criterion or criteria are set for evaluation of the motion by the at least one computing device.

In some embodiments, the pelvic sensor at least comprises an accelerometer and/or a gyroscope.

The accelerometer lets measure inclination and/or translation of the pelvic floor of the person whereas the gyroscope lets measure rotation and orientation of the pelvic floor. Any of these parameters may have to be evaluated for the determination of correct execution of the predetermined physical exercise.

In some embodiments, the at least one first criterion comprises one or more of: at least one body member exceeding a predetermined orientation or being within a predetermined orientation range, at least one body member exceeding a predetermined acceleration or being within a predetermined acceleration range, at least one body member exceeding a predetermined angular velocity or being within a predetermined angular velocity range, and at least one body member being still or substantially still with a predetermined orientation for a predetermined period of time.

By processing the first measurements, the computing device(s) establishes a motion tracking sequence of at least the tracked body members and compares it with the criteria associated with the concerned body members and movements to be performed by the person to assess whether the person is doing the physical exercise correctly. An exemplary evaluation procedure is disclosed in WO2019243438A1, which is entirely herein incorporated by reference.

In some embodiments, the predetermined physical exercise further comprises lack of motion of at least one body member of the person; the first measurements further measure motion of the at least one body member; and the determination of whether the person has performed the predetermined physical exercise further comprises evaluating the processed first measurements to determine whether the lack of motion of the at least one body member fulfills at least one further criterion of the predetermined set of criteria, where the at least one further criterion is associated with lack of motion of the at least one body member.

One or several body members may have to stay motionless during part or the entirety of the physical exercise. The computing device(s) is capable of monitoring that the body member(s) in fact stay motionless by processing the measurements and evaluating them with respect to the one or more further (e.g., third, fourth, etc.) criteria. For that, the computing device(s) compares the motion tracking sequence it derives from the processed measurements with the criterion or criteria associated with motionless body members.

In some embodiments, the method further comprises commanding provision of one or more user perceptible signals at least representative of the predetermined physical exercise. The perceptible signal(s) may additionally or alternatively include guidance on how to perform the predetermined physical exercise.

The person does the predetermined physical exercise after being instructed by the at least one computing device.

In some embodiments, a finite-state machine comprises or codifies the predetermined set of criteria, and determination of whether the person has performed the predetermined physical exercise according to the predetermined set of criteria comprises running the finite-state machine.

The at least one computing device may conduct all the evaluations and, thus, make the determination by running the state machine.

Multiple implementations are possible; for instance, transition between the several states can be based on flags used as inputs of the finite-state machine. The computing device(s) digitally provides flags indicative of fulfillment or non-fulfillment of a respective criterion of the predetermined set of criteria as part of the evaluation of the measurements. The flags therefore make the finite-state machine transition between the states as new flags are provided. The finite-state machine may arrive at a state indicative of incorrect execution of the predetermined physical exercise even before the person has finished doing the physical exercise if the measurements are provided to the computing device(s) on-the-fly and processed accordingly, and/or after completion of the physical exercise by the person, in which case the finite-state machine is made transition to that incorrect execution state at that time.

A finite-state machine eases the implementation and assessment of multiple criteria, even more so in those embodiments in which the computing device(s) evaluates criterion/criteria associated with sequence in which motion of the one or more body members and the at least one of lengthening and contracting of a pelvic floor muscle must occur during performance of the predetermined physical exercise. It is noted that a repetition of a physical exercise may involve two or more criteria, e.g., five, ten, fifteen or even more criteria to be met.

A second aspect of the disclosure relates to a data processing device or system comprising: at least one processor adapted to perform a method according to the first aspect.

The device or system may be part of a motion tracking system configured to automatically and digitally supervise the performance of physical exercises, particularly at least pelvic floor exercises that involve lengthening and/or contracting of the pelvic floor muscle. Further, the device or system might likewise cause the provision of instructions to the person for notifying of incorrect exercising and/or even how to correctly exercise whenever the person fails to correctly perform the exercise.

The device or system may comprise at least one memory and computer program code stored therein with instructions that, when run by the at least one processor, cause the device or system to perform the steps, namely the device or system: processes first measurements taken at least while a person performs a predetermined physical exercise, where: the predetermined physical exercise comprises both movement of one or more body members of the person and at least one of lengthening and contracting of a pelvic floor muscle of the person; and the first measurements are taken by at least one motion tracking device arranged on the person to measure motion of at least the one or more body members, and/or at least one optical sensor arranged to capture images of at least a portion of the person to measure motion of at least the one or more body members; processes second measurements taken at least while the person performs the predetermined physical exercise, where the second measurements are taken by a pelvic sensor; and determines whether the person has performed the predetermined physical exercise according to a predetermined set of criteria associated with both the predetermined physical exercise by: evaluating the processed first measurements to determine whether motion of the one or more body members fulfills at least one first criterion of the predetermined set of criteria, where the at least one first criterion is associated with motion of the one or more body members; and evaluating the processed second measurements to determine whether the pelvic floor muscle lengthens and/or contracts such that it fulfills at least one second criterion of the predetermined set of criteria, where the at least one second criterion is associated with at least one of lengthening and contracting of a pelvic floor muscle; in this sense, the device or system determines that the person has correctly performed the predetermined physical exercise when all criteria of the predetermined set of criteria have been fulfilled.

A third aspect of the disclosure relates to a device or system comprising: means adapted to execute the steps of a method according to the first aspect.

A fourth aspect of the disclosure relates to a motion tracking system comprising: at least one motion tracking device and/or at least one optical sensor; a pelvic sensor; and a device or system according to the second aspect or a device according to the third aspect.

A fifth aspect of the disclosure relates to a computer program product that has instructions which, when executed by at least one computing device like e.g. at least one device according the second aspect or third aspect, cause the at least one computing device to carry out the steps of a method according to the first aspect.

In some embodiments, the computer program product is embodied on a non-transitory computer-readable medium or a computer-readable data carrier has the computer program product stored thereon.

A sixth aspect of the disclosure relates to a data carrier signal carrying a computer program product according to the fifth aspect.

A seventh aspect of the disclosure relates to the use of a method, device, system, computer program or data carrier signal as described in the previous aspects for physical rehabilitation of a person.

Similar advantages as those set out with reference to the first aspect of the disclosure are likewise applicable to the remaining aspects of the disclosure. Another aspect of the present disclosure provides a non-transitory computer readable medium comprising machine executable code that, upon execution by one or more computer processors, implements any of the methods above or elsewhere herein.

Another aspect of the present disclosure provides a system comprising one or more computer processors and computer memory coupled thereto. The computer memory comprises machine executable code that, upon execution by the one or more computer processors, implements any of the methods above or elsewhere herein.

Additional aspects and advantages of the present disclosure will become readily apparent to those skilled in this art from the following detailed description, wherein only illustrative embodiments of the present disclosure are shown and described. As will be realized, the present disclosure is capable of other and different embodiments, and its several details are capable of modifications in various obvious respects, all without departing from the disclosure. Accordingly, the drawings and description are to be regarded as illustrative in nature, and not as restrictive.

### INCORPORATION BY REFERENCE

All publications, patents, and patent applications mentioned in this specification are herein incorporated by reference to the same extent as if each individual publication, patent, or patent application was specifically and individually indicated to be incorporated by reference. To the extent publications and patents or patent applications incorporated by reference contradict the disclosure contained in the specification, the specification is intended to supersede and/or take precedence over any such contradictory material.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings (also "Figure" and "FIG." herein), of which:
FIG. 1 shows a system, in accordance with some embodiments;
FIG. 2 shows a person doing an exemplary pelvic floor exercise to be supervised by methods, devices and systems, in accordance with some embodiments;
FIG. 3 shows a method, in accordance with some embodiments;
FIG. 4 shows a method, in accordance with some embodiments;
FIG. 5 shows an exemplary finite-state machine diagram, in accordance with some embodiments;
FIG. 6 shows an example graphical user interface (GUI), in accordance with some embodiments; and
**FIG. 7** shows a computer system that is programmed or otherwise configured to implement methods provided herein.

### DETAILED DESCRIPTION

While various embodiments of the invention have been shown and described herein, it will be obvious to those skilled in the art that such embodiments are provided by way of example only. Numerous variations, changes, and substitutions may occur to those skilled in the art without departing from the invention. It should be understood that various alternatives to the embodiments of the invention described herein may be employed.

Whenever the term "at least," "greater than," or "greater than or equal to" precedes the first numerical value in a series of two or more numerical values, the term "at least," "greater than" or "greater than or equal to" applies to each of the numerical values in that series of numerical values. For example, greater than or equal to 1, 2, or 3 is equivalent to greater than or equal to 1, greater than or equal to 2, or greater than or equal to 3.

Whenever the term "no more than," "less than," or "less than or equal to" precedes the first numerical value in a series of two or more numerical values, the term "no more than," "less than," or "less than or equal to" applies to each of the numerical values in that series of numerical values. For example, less than or equal to 3, 2, or 1 is equivalent to less than or equal to 3, less than or equal to 2, or less than or equal to 1.

### Overview

Disclosed is a method. The method may process first measurements taken at least while a person performs a predetermined physical exercise. The physical exercise may exercise the pelvic floor muscle. For example, the exercise may be a kegel or bridge exercise. The exercise may be performed sitting down, standing up, kneeling, or lying down (e.g., in a supine or prone position). An exercise may comprise one or more extensions or contractions of the pelvic floor muscle.

At least one of the first measurements or further measurements may be taken by the at least one optical sensor, and the method may further comprise processing, by the at least one computing device, the at least one of the first measurements or further measurements taken at least while the person performs the predetermined physical exercise. The optical sensor may comprise a camera configured to capture still images and/or video. The optical sensor may be configured to capture red-green-blue (RGB) images, YUV images, depth images, infrared images or video, or a combination thereof. Processing the first measurements (e.g., images or video) from the optical sensor may comprise one or more computer vision and/or machine learning techniques. For example, an ML-based system may generate learned representations of the optical, motion sensor, and pelvic sensor data. Then, these learned representations may be processed using one or more machine learning models, to generate one or more predictions related to whether a person's pose is correct, or whether positions or orientations of individual body members are correct, or both. For example, the measurements may be processed using one or more video-based object tracking or image-based object tracking algorithms. These algorithms may be computer vision algorithms. The computer vision algorithms may be machine learning (e.g., deep learning or neural network-based) algorithms or may be algorithms that do not involve machine learning. For example, processing the measurements may be performed using one or more pose estimation algorithms. The algorithms may comprise, for example, open pose, pose net, Blaze pose, deep pose, dense pose, deep cut models, or a combination thereof.

Determining whether the person has performed the predetermined physical exercise may further comprise evaluating the processed measurements of the at least one optical sensor to determine whether motion of one or more joints of the person fulfills at least one further criterion of the criteria. The predetermined physical exercise may comprise both movement of one or more body members of the person and at least one of lengthening or contracting of a pelvic floor muscle of the person. The predetermined physical exercise may further comprise a lack of motion of at least one body member of the person. The first measurements may further measure motion of the at least one body member. Determining whether the person has performed the predetermined physical exercise may further comprise evaluating the processed first measurements to determine whether the motion of the at least one body member fulfills at least one criterion of the criteria associated with lack of motion. The criteria may be associated with a position, orientation (e.g., angle), velocity (e.g., velocity associated with positional displacement or angular velocity not associated with positional displacement, or a combination thereof), acceleration, or jerk (e.g., change of acceleration) of one or more body members. The criteria may be associated with a position, orientation, velocity, or acceleration of one or more body members relative to one or more additional body members of the subject.

In some cases, the first measurements may be taken by at least one motion tracking device, at least one optical sensor, or both.

The first measurements may be at least taken by the at least one motion tracking device, wherein each motion tracking device of the at least one motion tracking device comprises an accelerometer and a gyroscope. The motion tracking device may also comprise a magnetometer or other inertial motion capture system, a mechanical motion capture system, or a stretch system. The at least one motion tracking device may be configured to be arranged on or in proximity to the person to measure motion of at least the one or more body members. For example, a motion tracking device may measure motion of at least one body member while contacted to the body member. The motion tracking device may be contacted to the body, but not the body member being measured. The motion tracking device may measure body motion without being contacted to the body.

The at least one optical sensor may be configured to capture images or video of the person to measure motion of at least the one or more body members.

In some cases, the criteria may comprise at least one first criterion associated with motion of the one or more body members and at least one second criterion associated with at least one of lengthening or contracting of the pelvic floor muscle. The at least one first criterion may comprise one or more of at least one body member exceeding a predetermined orientation or being within a predetermined orientation range, at least one body member exceeding a predetermined acceleration or being within a predetermined acceleration range, at least one body member exceeding a predetermined angular velocity or being within a predetermined angular velocity range, and at least one body member being still or substantially still with a predetermined orientation for a predetermined period of time.

Determining whether the person has performed the predetermined exercise may comprise evaluating the processed first measurements to determine whether the motion of the one or more body members fulfills the at least one first criterion of the criteria. Determining whether the person has performed the predetermined exercise may comprise evaluating the processed second measurements to determine whether the pelvic floor muscle at least lengthens or contracts such that it fulfills the at least one second criterion of the criteria.

Evaluating the processed second measurements to determine whether the pelvic floor muscle at least lengthens or contracts such that it fulfills the at least one second criterion of the criteria may comprise evaluating the processed second measurements to determine whether the pelvic floor muscle at least lengthens or contracts such that it fulfills the at least one second criterion of the criteria may comprise calculating at least one ratio of the pelvic floor muscle lengthening or contracting thereby calculating how much the person lengthened or contracted the pelvic floor muscle.

Determining whether the person has performed the predetermined physical exercise may further comprise evaluating the processed first and second measurements to determine whether the motion of the one or more body members and the at least one of lengthening or contracting of the pelvic floor muscle fulfill at least one third criterion of the criteria. The at least one third criterion may at least require that the at least one of lengthening or contracting of the pelvic floor muscle must occur during performance of the predetermined physical exercise.

The method may process second measurements taken at least while the person performs the predetermined physical exercise. The second measurements may be taken at least while the person performs the predetermined physical exercise. The second measurements may be taken by a pelvic sensor. A pelvic sensor may be a rectal or vaginal sensor. A pelvic sensor may be placed on an abdomen of the human subject. The pelvic sensor further may measure motion of a pelvic floor of the person. The determination of whether the person has performed the predetermined physical exercise further may comprise evaluating the processed second measurements to determine whether motion of the pelvic floor fulfills at least one further criterion of the criteria.

The method may next determine whether the person has performed the predetermined physical exercise based at least in part on the first measurements and the second measurements according to criteria associated with the predetermined physical exercise.

The method may further comprise providing, by the at least one computing device, one or more instructions or signals indicative of incorrect performance of the predetermined physical exercise when at least one criterion of the of criteria has not been fulfilled according to the determining step, transmitting, by the at least one computing device to another computing device, data representative of incorrect performance of the predetermined physical exercise when at least one criterion of the predetermined set of criteria has not been fulfilled, or modifying, by the at least one computing device, the criteria by disabling one or more criteria thereof, or by changing values of one or more criteria thereof. The one or more instructions or signals may be indicative of at least one of which criterion have not been fulfilled; and guidance on how to move the one or more body members or the pelvic floor muscle to fulfill the criterion that have not been fulfilled. A signal may be an audio, video, image, textual, haptic, or tactile cue, or a combination thereof. The system may provide instructions for the user to stop performance of an exercise or movement, correct performance of the exercise or movement, restart an exercise or movement, perform an alternative exercise or movement, or a combination thereof. A signal may provide stimulation to one or more body members to induce correction of a movement or exercise.

The method may further comprise processing, by the at least one computing device, a third measurement taken by the pelvic sensor while the person keeps the pelvic floor muscle relaxed, a fourth measurement taken by the pelvic sensor while the person lengthens the pelvic floor muscle, or a fifth measurement by the pelvic sensor while the person contracts the pelvic floor muscle; and calibrating, by the at least one computing device, the processed second measurements or at least a portion of the criteria based on the third measurement and at least one of the fourth measurement or the fifth measurement. The method may further comprise retrieving, by the at least one computing device, data associated with the person. The data may be representative of at least one of a relaxed state, a lengthened state, or a contracted state of the pelvic floor muscle of the person. The method may further comprise calibrating, by the at least one computing device, the processed second measurements or the at least one second criterion based on the data representative of at least one of the relaxed state, the lengthened state, or the contracted state of the pelvic floor muscle of the person.

### System

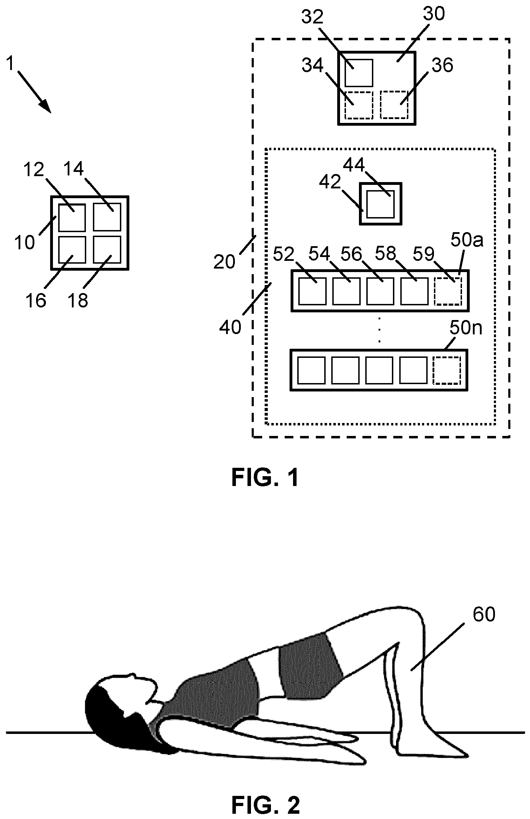

FIG. 1 shows a motion tracking system 1, in accordance with some embodiments. The system 1 may include at least one computing device 10, and a set of sensors 20 (shown with a dashed line for illustrative purposes only).

Each computing device 10 may include at least one processor 12, at least one memory 14, a communications module 16 at least for reception of data. The communications module 16 may likewise be adapted for transmission of data. The communications module 16, can be for wired or wireless communications. The at least one memory 14 may store instructions and/or a computer program that, upon execution by the at least one processor 12, cause the computing device(s) 10 to supervise the execution of physical exercises by a person. The computing device(s) 10 may be e.g. a tablet, a mobile phone, a personal computer, and field programmable gate array (FPGA), an application-specific integrated circuit (ASIC), etc.

Although not illustrated, the computing device(s) 10 may include further components, such as user presenting means (e.g., a screen, loudspeakers, etc.) and/or user input means (e.g. a touchscreen, a keyboard, etc.). For example, the computing device(s) 10 might present to a user how to arrange motion tracking devices 50a-50n on the body, and/or which physical exercise(s) the user shall do for instance during a physical rehabilitation procedure automatically supervised by the motion tracking system 1, and/or feedback concerning how user has done the physical exercise(s).

The computing device may provide a user interface. The user interface may be a graphical user interface (GUI),

The set of sensors 20 may include a pelvic sensor 30, and: at least one optical sensor 42, and/or one or more motion tracking devices 50a-50n. Therefore, from a group 40 represented with a dotted line for illustrative purposes only, one or both type of sensors 42, 50a-50n is/are part of the set of sensors 20 of the system 1.

The pelvic sensor 30 may measure a magnitude that is representative of the lengthening and/or contracting of the pelvic floor muscle, for example but without limitation, pressure in the pelvis or in the vagina, force applied by the pelvis, electrical activity from the pelvic floor muscle, etc. The pelvic sensor 30 may be any that enables to measure a magnitude representative of the lengthening and contracting of the pelvic floor muscle. Some non-limiting examples of a pelvic sensor 30 are: an external sensor based on a muscular force sensor, or based on a beam and a sensor for sensing a movement of and/or a force exerted on the beam like, for instance, that described in US20200384311A1; an internal sensor such as an intravaginal sensor, or an anorectal plug sensor like, for instance, that described in US8147429B2, etc.

The pelvic sensor 30 includes a communications module 32 at least for transmission of data, the module 32 preferably being a wireless communications module (e.g., capable of communicating via Wi-Fi, Bluetooth, radio transmission, cellular network, or another wireless communications method). Optionally, the pelvic sensor 30 also includes an inertial measurement unit 34 (shown with dashed lines to represent that the unit 34 is included in some embodiments) that preferably includes at least one of an accelerometer and a gyroscope, more preferably both sensing devices; and/or a vibrator device 36 (also shown with dashed lines for the sake of clarity).

Each optical sensor 42 includes a communications module 44 that is preferably a wireless communications module. Although not represented, it is noted that the optical sensor(s) 42 is configured to capture images or video and includes at least one processor and at least one memory to that end. In some embodiments, the at least one optical sensor 42 is embodied in the at least one computing device 10.

Each motion tracking device of the one or more motion tracking devices 50a-50n includes an inertial measurement unit 52, at least one processor 54, at least one memory 56, and a communications module 58 that is preferably a wireless communications module. The inertial measurement unit 52 preferably at least includes an accelerometer and a gyroscope, but may include other sensing devices as well, such as a magnetometer. In addition to the measurements, the inertial measurement unit 52 may provide, the at least one processor 54 may run a sensor fusion algorithm whereby the different measurements provided are combined to provide enhanced measurements. For example, the sensor fusion algorithm may produce a weighted sum or score from the different sensor measurements. The sensor fusion algorithm may comprise a machine learning model. The machine learning model may, for example, generate one or more representations (e.g., via dimensionality reduction or representation learning) of data from each type of sensor used. The representations may be configured to be easily combined or summed. The system may then provide the representations to a computational layer to generate a prediction or score from the learned representations. The machine learning model may comprise, for example, a neural network (e.g., a convolutional neural network or a recurrent neural network), a tree algorithm (e.g., random forests, Gradient Boosted Trees, or AdaBoost), support vector machines, logistic regression, or another algorithm. Although not illustrated, each motion tracking device 50a-50n includes an attaching device for attachment to the person; the attaching device might be any known in the art, e.g. straps, Velcro, etc. The motion tracking device(s) 50a-50n might also include a vibrator device 59.

FIG. 2 shows a person 60 doing an exemplary pelvic floor exercise to be supervised by methods, devices and systems, in accordance with some embodiments.

In an example exercise, the person 60 may lie on the floor in a supine position (e.g., face upward). As part of the pelvic floor exercise, the person 60 may raise the pelvis and exercise the pelvic floor muscle whilst the lower legs are preferably as perpendicular to the floor as possible.

It is noted that, apart from the exercise illustrated in FIG. 2, any other pelvic floor exercise is possible within and is encompassed by the scope of the present disclosure. For example, a pelvic floor exercise may be performed from a person is in a prone position (e.g., face down), seated, standing, or kneeling. A pelvic floor exercise may comprise movement or repositioning of at least one, at least two, or at least three body members. A pelvic floor exercise may comprise a single movement or repeated movements. The system may analyze multiple repetitions, or multiple sets of repetitions, of one or more pelvic floor exercises. Pelvic floor exercises may comprise bridges, bird dogs, kegels (e.g., quick flick kegels), heel sides, marches, happy baby pose, diaphragmatic breathing, a combination thereof, or the like.

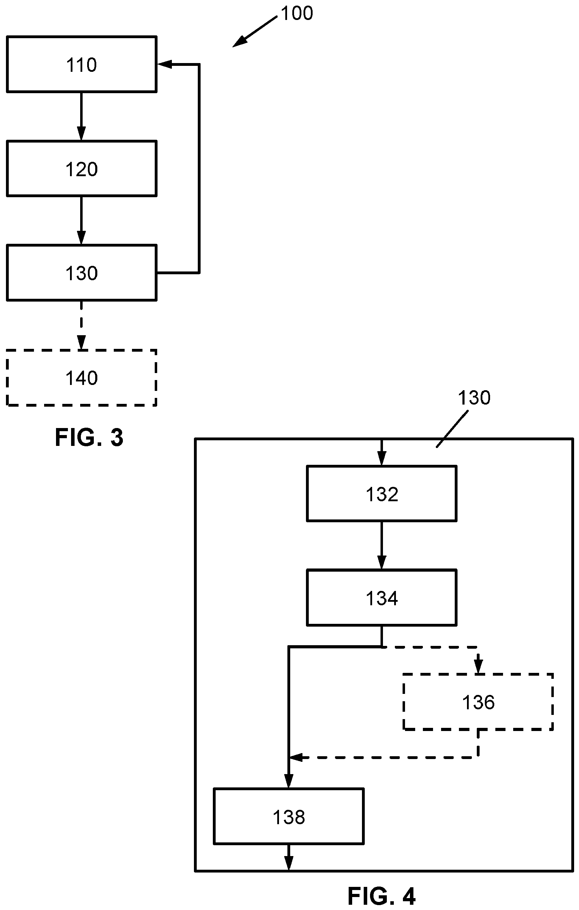

FIG. 3 shows a method 100, in accordance with some embodiments.

The method 100 may include a step whereby a computing device (or devices), like e.g., computing device 10 of FIG. 1, processes 110 first measurements that were taken at least while a person performed a predetermined physical exercise. The computing device may automatically supervise how the person performed the physical exercise. This way, the computing device may have data indicative of the predetermined physical exercise that the person performs; in this sense, in some embodiments the method 100 also includes a previous step whereby the computing device causes to present the predetermined physical exercise to the person by way of user presenting means. The predetermined physical exercise may be a pelvic floor exercise that involves motion of one or more body members of the person and lengthening or contracting (or both) of a pelvic floor muscle of the person.

The first measurements to be processed 110 may be measurements provided by one or more optical sensors, e.g., optical sensor 42 of FIG. 1, arranged in such a way that the images taken, which can be either still photos or video footage, may at least include part of the body of the person performing the physical exercise for motion tracking of the body members. Additionally or alternatively, the measurements may be provided by one or more motion tracking devices, like e.g. motion tracking device(s) 50a-50n of FIG. 1, each arranged on a body member of the person for motion tracking; the measurements may be those provided by the sensing devices and/or those provided by a sensor fusion algorithm.

Whenever the first measurements at least include measurements taken by the motion tracking device(s), the computing device may have data indicative of a correspondence between each motion tracking device and the location thereof on the body of the person, for example: a first motion tracking device having a first identification is arranged on the left thigh of the person, a second motion tracking device having a second identification is arranged on the right thigh of the person, and a third motion tracking device may have a third identification is arranged on the hip. In some cases, the correspondence may not only identify the body member where the motion tracking device is at, but also the positioning on that body member, e.g. at the middle point of the segment of the body member, at the front part of the body member, etc. To this end, the method 100 may also include a step whereby the person arranges the motion tracking device(s) thereon, and manually introduces in the computing device where each motion tracking device has been arranged; or a calibration process may be conducted whereby the person performs some predetermined movement(s) that the computing device indicates to infer, based on the measurements of each motion tracking device, where the motion tracking device is considering the particularities of the predetermined movement(s); or the computing device indicates on which particular body member each particular motion tracking device shall be arranged. Tracked body members may depend upon the physical exercise to be performed, therefore data representative of which body members must be tracked is stored in a memory of the computing device or may be gathered from a remote device, for instance a server; the tracked body members may be defined by any user, including a personal trainer or physical therapist that prescribes the exercise to the person. Namely, any of the above data can be transmitted to and/or received from another computing device using a communications module of the computing device. For example, a therapist may be able to receive the feedback at a computing device in a hospital to monitor the evolution of the person. Based on the feedback received, the therapist may adjust the difficulty of the physical exercise(s), the number of repetitions thereof, prescribe new physical exercises, etc. so that the person may further exercise with automatic digital supervision.

The computing device may provide a motion tracking sequence at least for the body members whose motion is tracked by processing 110 the first measurements. If some body members are not actually tracked with the first measurements, the motion tracking sequence may include additional body members whose motion can be derived having regard joints in common with tracked body members and biomechanical constraints that exist in people.

The method 100 may include a further step whereby the computing device may process 120 second measurements that were taken at least while the person performed the predetermined physical exercise.

The second measurements to be processed 120 may be measurements provided by a pelvic sensor, like e.g. pelvic sensor 30 of FIG. 1. The second measurements may be of any magnitude that can be used to derive changes in length of the pelvic floor muscle, which the computing device may derive during the processing 120.

It is noted that the set of sensors take the measurements of the processing steps 110, 120 at least while the person may perform the predetermined physical exercise, and those measurements are transmitted to the computing device via e.g. a wireless or wired communications connection; any or both of the provision and transmission of the measurements may be part of the method in some embodiments.

Further, even if the processing 110 of the first measurements is shown preceding the processing 120 of the second measurements, this may not necessarily represent the actual sequence of these steps. The processing 110 of the first measurements may indeed take place before the processing 120 of the second measurements, or the processing 120 of the second measurements precede the processing 110 of the first measurements, or both might take place in parallel either simultaneously or with some delay between the processing 110, 120 of the first and second measurements.

The method 100 may include a further step whereby the computing device determines 130 whether the person has performed the predetermined physical exercise, based at least in part on the first measurements and the second measurements. To that end, each predetermined physical exercise has criteria or constraints related to motion of one or more specific body members that are to be considered for determination of how well the physical exercise is performed. The criteria may include at least one criterion related to motion of one or more body members, and at least one criterion related to the lengthening and/or contracting of the pelvic floor muscle. The criteria may be defined in accordance with the particulars of the person who performs the physical exercise, thereby making the criteria tailored to the physical condition of the person. Both the criteria and the physical exercise itself may be defined by any user, including a personal trainer or physical therapist that prescribes the exercise to the person.

With the measurements as processed 110, 120, the computing device evaluates whether the motion of the body member(s) and the changes in length of the pelvic floor muscle meet the criteria. When both meet all respective criteria, the computing device determines that the physical exercise has been correctly executed, and not correctly executed otherwise.

Since physical exercises may include a number of repetitions in each of which the person has to move the body members according to a predetermined movement associated with the exercise (and, preferably, also associated with the person as explained before), and lengthen and/or contract the pelvic floor muscle according to a predetermined change in length associated with the exercise (and, preferably, also associated with the person), upon making the determination 130 for a given repetition of the physical exercise, the method 100 is repeated with the processing 110, 120 of further measurements taken in a different repletion and with the determination 130. Alternatively, a different physical exercise may have to be performed, in which case the method 100 is repeated with different criteria to be met in the determination 130; moreover, the motion of other body members might have to be tracked, in which case the optical sensor(s) and/or the motion tracking device(s) may have to be rearranged for capturing the motion of those other body members.

As represented with dashed lines for the sake of clarity only, the method 100 may further include a step whereby the computing device causes to present 140 an outcome of the determination 130 made, or feedback to the person for improving the execution of the physical exercise whenever the execution has been determined to be incorrect.

FIG. 4 shows part of a method in accordance with embodiments. Particularly, FIG. 4 shows a determining step 130 as described in relation to FIG. 3 in more detail.

The determining step 130 may include a step whereby the computing device (or devices) evaluates 132 the processed 110 first measurements to determine whether motion of the body member(s) fulfills the criterion or criteria related to the motion of body members.

The determining step 130 also may include a step whereby the computing device evaluates 134 the processed 120 second measurements to determine whether the changes in length (if any were made by the person) of the pelvic floor muscle fulfill the criterion or criteria related to the lengthening and/or contracting of the pelvic floor muscle.

The determining step 130 also may include a step whereby the computing device makes the determination 138 about whether the person did perform the predetermined physical exercise correctly if each evaluation 132, 134, 136 conducted is such that all the related criterion or criteria have been met.

As represented with dashed lines for the sake of clarity only, the determining may further include a further step, prior to making the determination 138, whereby the computing device evaluates 136 some of the processed 110 measurements or some other processed measurements to establish if further criterion or criteria related to the physical exercise is/are also met. By way of example, the further criterion or criteria may be associated with the order in time in which the motion and the changes in length of the pelvic floor muscle have occurred (and, optionally, how much motion and/or change in length there have/has been at the different time instants during performance of the physical exercise), and/or associated with the motion of joints of the person. If any such additional evaluation or evaluations 136 exist, while making the determination 138 each additional evaluation 136 is considered.

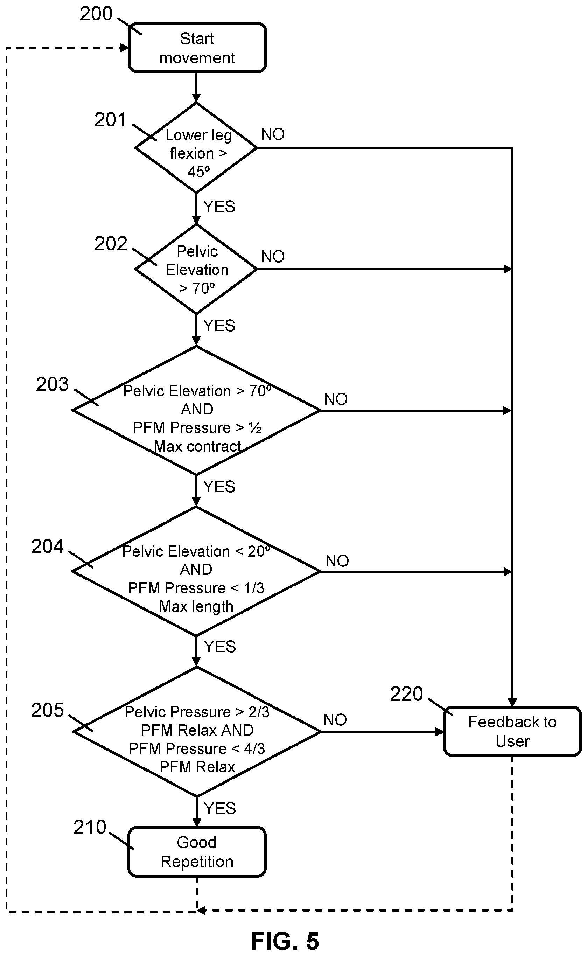

FIG. 5 shows an exemplary finite-state machine diagram, related to an exercise for e.g., a physical rehabilitation procedure, that can be implemented in some embodiments for supervision (e.g., monitoring and/or correction) of the exercise execution.

The exercise can be divided into a plurality of repetitions. At the beginning of each repetition, the movement that the person must do is started 200. Measurements related to the motion of the person and related to the length of the pelvic floor muscle may start to be provided when the movement is started 200, for instance by way of a command (with input means like, e.g. a microphone, a keyboard, a tactile screen, a computer mouse, a voice command, a physical gesture (e.g., a hand movement) etc.), or have started to be provided before the movements starts 200.

A first criterion 201 related to motion that a computing device checks for fulfillment is whether a lower leg of the person has flexed more than 45°. If the first criterion 201 is not fulfilled after some time (e.g. based on a predetermined time threshold), or if the trend of the movement of the lower leg is to reduce the flexion, the exercising by the person is determined as not being correct. In some cases, a criterion may be adjusted based at least in part on a person's movement. For example, a disabled or differently abled person may not be able to perform one or more movements of an exercise, or may not be able to perform an exercise with full range of motion. The system may be able to detect one or more limitations of motion of the subject and adjust the criterion accordingly. In the preceding example, if the person cannot flex more than 30°, the system may automatically adjust the criterion to check for fulfillment when the lower leg of the person has flexed more than 30°.

If fulfilled, a subsequent criterion 202 must be fulfilled: the pelvis is elevated beyond an angle of 70°. When the criterion 202 is not fulfilled after some time, the exercise is deemed not to be correctly performed, whereas if the criterion 202 is fulfilled the finite-state machine is advanced to another check with two criteria 203: the pelvic is still elevated beyond the angle of 70°, and the pressure of the pelvic floor muscle (i.e. PFM) is at least 50% of the maximum contraction of the PFM associated with the person. The maximum contraction may be predetermined, but is preferably determined by conducting a calibration procedure first.

When at least one of the two criteria 203 is not fulfilled after some time, the exercise is deemed not to be correctly performed, whereas if both criteria 203 are fulfilled the finite-state machine is advanced to subsequent double criterion 204 to be fulfilled: the pelvis is elevated below an angle of 20°, and the pressure of the PFM is less than one third of the maximum length. Namely, these additional criteria 204 are for the person to start the return to the original posture at the beginning.

If both criteria 204 are fulfilled, a last criterion 205 to be fulfilled requires that the pressure of the PFM is at least two thirds of the pressure of the PFM in the relaxed state associated with the person (again, predetermined or calibrated value of pressure) and less than four thirds of said pressure.

When the criteria are fulfilled according to this manner, the computing device determines that the repetition has been correctly executed 210. When not, the computing device may command, like in the present case, the provision of feedback 220 for the person to realize that there has been an incorrect execution of the movement.

It is noted that in this exemplary finite-state machine, the temporal sequence in which the criteria 201-205 are to be fulfilled is considered, but in other examples the temporal sequence may not be considered during evaluation of the exercising of the person.

Subsequently, as illustrated with dashed arrow lines, after the repetition has been correctly or incorrectly executed, further repetitions can be supervised again from the start 200 thereof, or repetitions of a different exercise can start to be supervised.

As it can be appreciated in the illustrated diagram, the different checks can be of a single criterion or of multiple criteria (two, three, or even more criteria at once). Although AND clauses have been indicated as part of some criteria 203-205, in other examples OR clauses could also be provided for setting alternative criteria whereby the fulfillment of one or several criteria is sufficient for clearing the criteria and advancing to further criteria, if any.

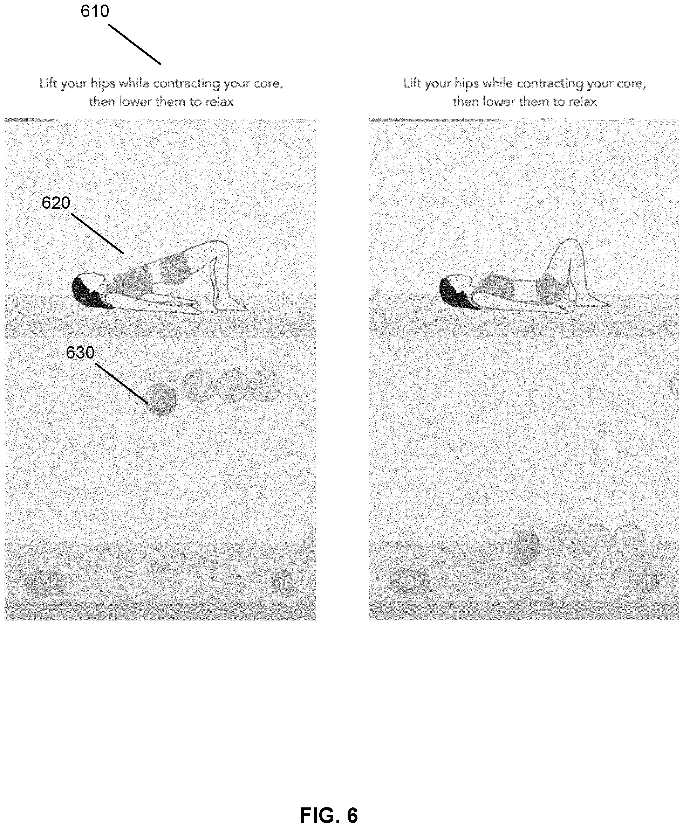

FIG. 6 illustrates an example graphical user interface (GUI) 600, in accordance with some embodiments. The GUI 600 may provide visual cues to assist a user with performing one or more exercises (e.g., pelvic floor exercises). The exercises may comprise isolated pelvic movements or may combine functional and pelvic exercises, or a combination thereof. The visual cues may comprise audio, video, or text presentations, or a combination thereof. The visual cues may include an animation of a subject body performing an exercise or movement and one or more visual representations of the position and/or orientation of the pelvic floor muscle and/or pelvic floor sensor. The visual cues may also comprise text instructions for performing an exercise or movement. For example, GUI presentation 600 may provide textual instructions 610 "Lift your hips while contracting your core, then lower them to relax" above a diagram image 620 of a subject performing the action associated with the text, above a diagram image 630 of a position and/or orientation of the pelvic floor muscle. A next screencap may display the same text alongside an image of a different position in the exercise or movement along with a corresponding position and orientation of the pelvic floor. In some cases, the text and visual cues may be displayed in a different orientation (e.g., on separate pages or screens, differently arranged in space, or in a temporal sequence rather than displayed simultaneously).

### Particular Implementations

Disclosed is a method. The method may process first measurements taken at least while a person performs a predetermined physical exercise. At least one of the first measurements or further measurements may be taken by the at least one optical sensor, and the method may further comprise processing, by the at least one computing device, the at least one of the first measurements or further measurements taken at least while the person performs the predetermined physical exercise. Determining whether the person has performed the predetermined physical exercise may further comprise evaluating the processed measurements of the at least one optical sensor to determine whether motion of one or more joints of the person fulfills at least one further criterion of the criteria. The predetermined physical exercise may comprise both movement of one or more body members of the person and at least one of lengthening or contracting of a pelvic floor muscle of the person. The predetermined physical exercise may further comprise lack of motion of at least one body member of the person. The first measurements may further measure motion of the at least one body member. Determining whether the person has performed the predetermined physical exercise may further comprise evaluating the processed first measurements to determine whether the motion of the at least one body member fulfills at least one criterion of the criteria associated with lack of motion. The first measurements may be taken by at least one motion tracking device, at least one optical sensor, or both. The first measurements may be at least taken by the at least one motion tracking device, wherein each motion tracking device of the at least one motion tracking device comprises an accelerometer and a gyroscope. The at least one motion tracking device may be configured to be arranged on the person to measure motion of at least the one or more body members. The at least one optical sensor may be configured to capture images or video of the person to measure motion of at least the one or more body members. The criteria may comprise at least one first criterion associated with motion of the one or more body members and at least one second criterion associated with at least one of lengthening or contracting of the pelvic floor muscle. The at least one first criterion may comprise one or more of at least one body member exceeding a predetermined orientation or being within a predetermined orientation range, at least one body member exceeding a predetermined acceleration or being within a predetermined acceleration range, at least one body member exceeding a predetermined angular velocity or being within a predetermined angular velocity range, and at least one body member being still or substantially still with a predetermined orientation for a predetermined period of time. Determining whether the person has performed the predetermined exercise may comprise evaluating the processed first measurements to determine whether the motion of the one or more body members fulfills the at least one first criterion of the criteria. Determining whether the person has performed the predetermined exercise may comprise evaluating the processed second measurements to determine whether the pelvic floor muscle at least lengthens or contracts such that it fulfills the at least one second criterion of the criteria. Determining whether the person has performed the predetermined physical exercise may further comprise evaluating the processed first and second measurements to determine whether the motion of the one or more body members and the at least one of lengthening or contracting of the pelvic floor muscle fulfill at least one third criterion of the criteria. The at least one third criterion at least requires that the at least one of lengthening or contracting of the pelvic floor muscle must occur during performance of the predetermined physical exercise. Evaluating the processed second measurements to determine whether the pelvic floor muscle at least lengthens or contracts such that it fulfills the at least one second criterion of the criteria comprises: Evaluating the processed second measurements to determine whether the pelvic floor muscle at least lengthens or contracts such that it fulfills the at least one second criterion of the criteria may comprise calculating at least one ratio of the pelvic floor muscle lengthening or contracting thereby calculating how much the person lengthened or contracted the pelvic floor muscle. The method may process second measurements taken at least while the person performs the predetermined physical exercise. The second measurements may be taken at least while the person performs the predetermined physical exercise. The second measurements may be taken by a pelvic sensor. The pelvic sensor further may measure motion of a pelvic floor of the person. The determination of whether the person has performed the predetermined physical exercise further comprises evaluating the processed second measurements to determine whether motion of the pelvic floor fulfills at least one further criterion of the criteria. The method may determine whether the person has performed the predetermined physical exercise based at least in part on the first measurements and the second measurements according to criteria associated with the predetermined physical exercise. The method may further comprise providing, by the at least one computing device, one or more instructions or signals indicative of incorrect performance of the predetermined physical exercise when at least one criterion of the of criteria has not been fulfilled according to the determining step, transmitting, by the at least one computing device to another computing device, data representative of incorrect performance of the predetermined physical exercise when at least one criterion of the predetermined set of criteria has not been fulfilled, or modifying, by the at least one computing device, the criteria by disabling one or more criteria thereof, or by changing values of one or more criteria thereof. The one or more instructions or signals may be indicative of at least one of which criterion have not been fulfilled; and guidance on how to move the one or more body members or the pelvic floor muscle to fulfill the criterion that have not been fulfilled. The method may further comprise processing, by the at least one computing device, a third measurement taken by the pelvic sensor while the person keeps the pelvic floor muscle relaxed, a fourth measurement taken by the pelvic sensor while the person lengthens the pelvic floor muscle, or a fifth measurement by the pelvic sensor while the person contracts the pelvic floor muscle; and calibrating, by the at least one computing device, the processed second measurements or at least a portion of the criteria based on the third measurement and at least one of the fourth measurement or the fifth measurement. The method may further comprise retrieving, by the at least one computing device, data associated with the person, wherein the data are representative of at least one of a relaxed state, a lengthened state, or a contracted state of the pelvic floor muscle of the person. The method may further comprise calibrating, by the at least one computing device, the processed second measurements or the at least one second criterion based on the data representative of at least one of the relaxed state, the lengthened state, or the contracted state of the pelvic floor muscle of the person.

In this text, the terms first, second, third, further, etc. have been used herein to describe several devices, elements or parameters, it will be understood that the devices, elements or parameters should not be limited by these terms since the terms are only used to distinguish one device, element or parameter from another. For example, the first measurements could as well be named second measurements, and the second measurements could be named first measurements without departing from the scope of this disclosure.

In this text, the term "includes", "comprises" and its derivations (such as "including", "comprising", etc.) should not be understood in an excluding sense, that is, these terms should not be interpreted as excluding the possibility that what is described and defined may include further elements, steps, etc.
On the other hand, the disclosure is obviously not limited to the specific embodiment(s) described herein, but also encompasses any variations that may be considered by any person skilled in the art (for example, as regards the choice of materials, dimensions, components, configuration, etc.), within the general scope of the invention as defined in the claims.

### Computer systems

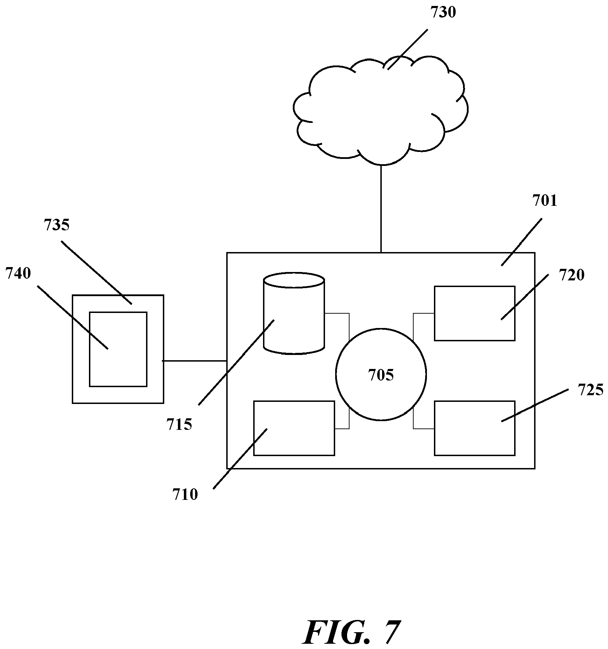

The present disclosure provides computer systems that are programmed to implement methods of the disclosure. **FIG. 7** shows a computer system 701 that is programmed or otherwise configured to determine whether a pelvic floor exercise is being performed correctly. The computer system 701 can regulate various aspects of monitoring of a pelvic floor exercise of the present disclosure, such as, for example, presenting instructions via a graphical user interface (GUI). The computer system 701 can be an electronic device of a user or a computer system that is remotely located with respect to the electronic device. The electronic device can be a mobile electronic device.

The computer system 701 includes a central processing unit (CPU, also "processor" and "computer processor" herein) 705, which can be a single core or multi core processor, or a plurality of processors for parallel processing. The computer system 701 also includes memory or memory location 710 (e.g., random-access memory, read-only memory, flash memory), electronic storage unit 715 (e.g., hard disk), communication interface 720 (e.g., network adapter) for communicating with one or more other systems, and peripheral devices 725, such as cache, other memory, data storage and/or electronic display adapters. The memory 710, storage unit 715, interface 720 and peripheral devices 725 are in communication with the CPU 705 through a communication bus (solid lines), such as a motherboard. The storage unit 715 can be a data storage unit (or data repository) for storing data. The computer system 701 can be operatively coupled to a computer network ("network") 730 with the aid of the communication interface 720. The network 730 can be the Internet, an internet and/or extranet, or an intranet and/or extranet that is in communication with the Internet. The network 730 in some cases is a telecommunication and/or data network. The network 730 can include one or more computer servers, which can enable distributed computing, such as cloud computing. The network 730, in some cases with the aid of the computer system 701, can implement a peer-to-peer network, which may enable devices coupled to the computer system 701 to behave as a client or a server.

The CPU 705 can execute a sequence of machine-readable instructions, which can be embodied in a program or software. The instructions may be stored in a memory location, such as the memory 710. The instructions can be directed to the CPU 705, which can subsequently program or otherwise configure the CPU 705 to implement methods of the present disclosure. Examples of operations performed by the CPU 705 can include fetch, decode, execute, and writeback.

The CPU 705 can be part of a circuit, such as an integrated circuit. One or more other components of the system 701 can be included in the circuit. In some cases, the circuit is an application specific integrated circuit (ASIC).

The storage unit 715 can store files, such as drivers, libraries and saved programs. The storage unit 715 can store user data, e.g., user preferences and user programs. The computer system 701 in some cases can include one or more additional data storage units that are external to the computer system 701, such as located on a remote server that is in communication with the computer system 701 through an intranet or the Internet.

The computer system 701 can communicate with one or more remote computer systems through the network 730. For instance, the computer system 701 can communicate with a remote computer system of a user (e.g., a mobile device). Examples of remote computer systems include personal computers (e.g., portable PC), slate or tablet PC's (e.g., Applet iPad, Samsung^{®} Galaxy Tab), telephones, Smart phones (e.g., Applet iPhone, Android-enabled device, Blackberry^{®}), or personal digital assistants. The user can access the computer system 701 via the network 730.

Methods as described herein can be implemented by way of machine (e.g., computer processor) executable code stored on an electronic storage location of the computer system 701, such as, for example, on the memory 710 or electronic storage unit 715. The machine executable or machine readable code can be provided in the form of software. During use, the code can be executed by the processor 705. In some cases, the code can be retrieved from the storage unit 715 and stored on the memory 710 for ready access by the processor 705. In some situations, the electronic storage unit 715 can be precluded, and machine-executable instructions are stored on memory 710.

The code can be pre-compiled and configured for use with a machine having a processer adapted to execute the code, or can be compiled during runtime. The code can be supplied in a programming language that can be selected to enable the code to execute in a pre-compiled or as-compiled fashion.

Aspects of the systems and methods provided herein, such as the computer system 701, can be embodied in programming. Various aspects of the technology may be thought of as "products" or "articles of manufacture" typically in the form of machine (or processor) executable code and/or associated data that is carried on or embodied in a type of machine readable medium. Machine-executable code can be stored on an electronic storage unit, such as memory (e.g., read-only memory, random-access memory, flash memory) or a hard disk. "Storage" type media can include any or all of the tangible memory of the computers, processors or the like, or associated modules thereof, such as various semiconductor memories, tape drives, disk drives and the like, which may provide non-transitory storage at any time for the software programming. All or portions of the software may at times be communicated through the Internet or various other telecommunication networks. Such communications, for example, may enable loading of the software from one computer or processor into another, for example, from a management server or host computer into the computer platform of an application server. Thus, another type of media that may bear the software elements includes optical, electrical and electromagnetic waves, such as used across physical interfaces between local devices, through wired and optical landline networks and over various air-links. The physical elements that carry such waves, such as wired or wireless links, optical links or the like, also may be considered as media bearing the software. As used herein, unless restricted to non-transitory, tangible "storage" media, terms such as computer or machine "readable medium" refer to any medium that participates in providing instructions to a processor for execution.

Hence, a machine readable medium, such as computer-executable code, may take many forms, including but not limited to, a tangible storage medium, a carrier wave medium or physical transmission medium. Non-volatile storage media include, for example, optical or magnetic disks, such as any of the storage devices in any computer(s) or the like, such as may be used to implement the databases, etc. shown in the drawings. Volatile storage media include dynamic memory, such as main memory of such a computer platform. Tangible transmission media include coaxial cables; copper wire and fiber optics, including the wires that comprise a bus within a computer system. Carrier-wave transmission media may take the form of electric or electromagnetic signals, or acoustic or light waves such as those generated during radio frequency (RF) and infrared (IR) data communications. Common forms of computer-readable media therefore include for example: a floppy disk, a flexible disk, hard disk, magnetic tape, any other magnetic medium, a CD-ROM, DVD or DVD-ROM, any other optical medium, punch cards paper tape, any other physical storage medium with patterns of holes, a RAM, a ROM, a PROM and EPROM, a FLASH-EPROM, any other memory chip or cartridge, a carrier wave transporting data or instructions, cables or links transporting such a carrier wave, or any other medium from which a computer may read programming code and/or data. Many of these forms of computer readable media may be involved in carrying one or more sequences of one or more instructions to a processor for execution.

The computer system 701 can include or be in communication with an electronic display 735 that comprises a user interface (UI) 740 for providing, for example, Instructions for correctly performing a pelvic floor exercise. Examples of UI's include, without limitation, a graphical user interface (GUI) and web-based user interface.

Methods and systems of the present disclosure can be implemented by way of one or more algorithms. An algorithm can be implemented by way of software upon execution by the central processing unit 705. The algorithm can, for example, perform pose estimation of a subject performing a pelvic floor exercise.

While preferred embodiments of the present invention have been shown and described herein, it will be obvious to those skilled in the art that such embodiments are provided by way of example only. It is not intended that the invention be limited by the specific examples provided within the specification. While the invention has been described with reference to the aforementioned specification, the descriptions and illustrations of the embodiments herein are not meant to be construed in a limiting sense. Numerous variations, changes, and substitutions will now occur to those skilled in the art without departing from the invention. Furthermore, it shall be understood that all aspects of the invention are not limited to the specific depictions, configurations or relative proportions set forth herein which depend upon a variety of conditions and variables. It should be understood that various alternatives to the embodiments of the invention described herein may be employed in practicing the invention. It is therefore contemplated that the invention shall also cover any such alternatives, modifications, variations or equivalents. It is intended that the following claims define the scope of the invention and that methods and structures within the scope of these claims and their equivalents be covered thereby.

Additional statements:
1. A method comprising:
   processing, by at least one computing device, first measurements taken at least while a person performs a predetermined physical exercise, wherein:
      the predetermined physical exercise comprises both movement of one or more body members of the person and at least one of lengthening and contracting of a pelvic floor muscle of the person; and
      the first measurements are taken by at least one motion tracking device arranged on the person to measure motion of at least the one or more body members, and/or at least one optical sensor arranged to capture images of at least a portion of the person to measure motion of at least the one or more body members;
   processing, by at least one computing device, second measurements taken at least while the person performs the predetermined physical exercise, wherein the second measurements are taken by a pelvic sensor;
   determining, by at least one computing device, whether the person has performed the predetermined physical exercise according to a predetermined set of criteria associated with the predetermined physical exercise by both:
      evaluating the processed first measurements to determine whether motion of the one or more body members fulfills at least one first criterion of the predetermined set of criteria, wherein the at least one first criterion is associated with motion of the one or more body members; and
      evaluating the processed second measurements to determine whether the pelvic floor muscle lengthens and/or contracts such that it fulfills at least one second criterion of the predetermined set of criteria, wherein the at least one second criterion is associated with at least one of lengthening and contracting of a pelvic floor muscle;
   wherein the at least one computing device determines that the person has correctly performed the predetermined physical exercise when all criteria of the predetermined set of criteria have been fulfilled.
2. The method of statement 1, further comprising:
   processing, by the at least one computing device, third, fourth and fifth measurements respectively taken while the person keeps the pelvic floor muscle relaxed, while the person lengthens the pelvic floor muscle, and while the person contracts the pelvic floor muscle, thereby providing data associated with the person representative of:
      the pelvic floor muscle of the person being in relaxed state;
      the pelvic floor muscle of the person being in lengthened state; and
      the pelvic floor muscle of the person being in contracted state;
   wherein the third, fourth and fifth measurements are taken by the pelvic sensor; and
   calibrating, by the at least one computing device, the processed second measurements or the at least one second criterion, wherein the calibration is based on:
      the data representative of the pelvic floor muscle being in the relaxed state; and
      at least one of: the data representative of the pelvic floor muscle being in the lengthened state; and
      the data representative of the pelvic floor muscle being in the contracted state.
3. The method of statement 1, further comprising:
   retrieving, by the at least one computing device, registered data associated with the person, wherein the registered data are representative of:
      the pelvic floor muscle of the person being in relaxed state;
      the pelvic floor muscle of the person being in lengthened state; and
      the pelvic floor muscle of the person being in contracted state;
   calibrating, by the at least one computing device, the processed second measurements or the at least one second criterion, wherein the calibration is based on:
      the data representative of the pelvic floor muscle being in the relaxed state; and
      at least one of:
         the data representative of the pelvic floor muscle being in the lengthened state; and
         the data representative of the pelvic floor muscle being in the contracted state.
4. The method of any one of statements 2-3, wherein the evaluation of the processed second measurements to determine whether the lengthening and/or contracting of the pelvic floor muscle fulfills the at least one second criterion comprises: calculating at least one ratio of the pelvic floor muscle lengthening and/or contracting thereby calculating how much the person lengthened and/or contracted the pelvic floor muscle based on the calibrated relaxed and the lengthened and/or contracted states thereof, wherein the at least one ratio is calculated based on:
   the data representative of the pelvic floor muscle being in the relaxed state; and
   the data representative of the pelvic floor muscle being in the lengthened state and/or the contracted state; and
   wherein the at least one second criterion comprises at least one lengthening and/or contracting ratio of the pelvic floor muscle.
5. The method of any one of statements 1-4, wherein the determination of whether the person has performed the predetermined physical exercise further comprises:
   evaluating the processed first and second measurements to determine whether the motion of the one or more body members and the lengthening and/or contracting of the pelvic floor muscle fulfill at least one third criterion of the predetermined set of criteria, wherein the at least one third criterion is at least associated with a sequence in which motion of the one or more body members and the at least one of lengthening and contracting of a pelvic floor muscle must occur during performance of the predetermined physical exercise.
6. The method of any one of statements 1-5, further comprising one or more of the following:
   commanding, by the at least one computing device, provision of one or more user perceptible signals at least representative of incorrect performance of the predetermined physical exercise when at least one criterion of the predetermined set of criteria has not been fulfilled according to the determining step;
   transmitting, by the at least one computing device to another computing device, data representative of incorrect performance of the predetermined physical exercise when at least one criterion of the predetermined set of criteria has not been fulfilled; and
   modifying, by the at least one computing device, the predetermined set of criteria by disabling one or more criteria thereof, and/or by changing values of one or more criteria thereof.
7. The method of statement 6, wherein the one or more user perceptible signals comprise indications of:
   which predetermined criterion or criteria have not been fulfilled; and/or
   guidance on how to move the one or more body members and/or the pelvic floor muscle to fulfill the predetermined criterion or criteria that have not been fulfilled.
8. The method of any one of statements 1-7, wherein:
   either:
      the first measurements are at least taken by the at least one optical sensor; or
      the method further comprises processing, by the at least one computing device, further measurements taken at least while the person performs the predetermined physical exercise, wherein the further measurements are taken by the at least one optical sensor;
   wherein the determination of whether the person has performed the predetermined physical exercise further comprises evaluating the processed measurements of the at least one optical sensor to determine whether motion of one or more joints of the person fulfills at least one further criterion of the predetermined set of criteria, wherein the at least one further criterion is associated with motion of the one or more joints.
9. The method of any one of statements 1-8, wherein the first measurements are at least taken by the at least one motion tracking device; wherein each motion tracking device of the at least one motion tracking device at least comprises an accelerometer and a gyroscope.
10. The method of any one of statements 1-9, wherein the pelvic sensor further measures motion of a pelvic floor of the person; wherein the determination of whether the person has performed the predetermined physical exercise further comprises evaluating the processed second measurements to determine whether motion of the pelvic floor fulfills at least one further criterion of the predetermined set of criteria, wherein the at least one further criterion is associated with motion of the pelvic floor.
11. The method of any one of statements 1-10, wherein the at least one first criterion comprises one or more of: at least one body member exceeding a predetermined orientation or being within a predetermined orientation range, at least one body member exceeding a predetermined acceleration or being within a predetermined acceleration range, at least one body member exceeding a predetermined angular velocity or being within a predetermined angular velocity range, and at least one body member being still or substantially still with a predetermined orientation for a predetermined period of time.
12. The method of any one of statements 1-11, wherein:
   the predetermined physical exercise further comprises lack of motion of at least one body member of the person;
   the first measurements further measure motion of the at least one body member; and
   the determination of whether the person has performed the predetermined physical exercise further comprises evaluating the processed first measurements to determine whether the lack of motion of the at least one body member fulfills at least one further criterion of the predetermined set of criteria, wherein the at least one further criterion is associated with lack of motion of the at least one body member.
13. A device or system comprising: means adapted to execute the steps of a method according to any one of statements 1-12.
14. A motion tracking system comprising:
   at least one motion tracking device and/or at least one optical sensor;
   a pelvic sensor; and
   a device or system according to statement 13.
15. A computer program product that has instructions which, when the program is executed by at least one computing device, cause the at least one computing device to carry out the steps of a method according to any one of statements 1-12.

## Claims

1. A computer-implemented method comprising:
processing, by at least one computing device, first measurements taken at least while a person performs a predetermined physical exercise, wherein the first measurements are taken by at least one motion tracking device, at least one optical sensor, or both;
processing, by the at least one computing device, second measurements taken at least while the person performs the predetermined physical exercise, wherein the second measurements are taken by a pelvic sensor; and
determining, by at least one computing device, whether the person has performed the predetermined physical exercise based on the first measurements and the second measurements according to criteria associated with the predetermined physical exercise.

2. The method of claim 1, wherein the predetermined physical exercise comprises both movement of one or more body members of the person and at least one of lengthening or contracting of a pelvic floor muscle of the person.

3. The method of any one of the preceding claims, wherein the at least one motion tracking device is configured to be arranged on the person to measure motion of at least the one or more body members, or wherein the at least one optical sensor is configured to capture images or video of the person to measure motion of at least the one or more body members.

4. The method of any one of the preceding claims, wherein the criteria comprises at least one first criterion associated with motion of the one or more body members and at least one second criterion associated with at least one of lengthening or contracting of the pelvic floor muscle.

5. The method of any one of the preceding claims, wherein determining whether the person has performed the predetermined exercise comprises evaluating the processed first measurements to determine whether the motion of the one or more body members fulfills the at least one first criterion of the criteria.

6. The method of any one of the preceding claims, wherein determining whether the person has performed the predetermined exercise comprises evaluating the processed second measurements to determine whether the pelvic floor muscle at least lengthens or contracts such that it fulfills the at least one second criterion of the criteria.

7. The method of any one of the preceding claims, wherein evaluating the processed second measurements to determine whether the pelvic floor muscle at least lengthens or contracts such that it fulfills the at least one second criterion of the criteria comprises: calculating at least one ratio of the pelvic floor muscle lengthening or contracting thereby calculating how much the person lengthened or contracted the pelvic floor muscle.

8. The method of any one of the preceding claims, wherein determining whether the person has performed the predetermined physical exercise further comprises:
evaluating the processed first and second measurements to determine whether the motion of the one or more body members and the at least one of lengthening or contracting of the pelvic floor muscle fulfill at least one third criterion of the criteria, wherein the at least one third criterion at least requires that the at least one of lengthening or contracting of the pelvic floor muscle must occur during performance of the predetermined physical exercise.

9. The method of any one of the preceding claims, wherein the at least one first criterion comprises one or more of:
at least one body member exceeding a predetermined orientation or being within a predetermined orientation range,
at least one body member exceeding a predetermined acceleration or being within a predetermined acceleration range,
at least one body member exceeding a predetermined angular velocity or being within a predetermined angular velocity range, and
at least one body member being still or substantially still with a predetermined orientation for a predetermined period of time.

10. The method of any one of the preceding claims, further comprising one or more of the following:
providing, by the at least one computing device, one or more instructions or signals indicative of incorrect performance of the predetermined physical exercise when at least one criterion of the of criteria has not been fulfilled according to the determining step;
transmitting, by the at least one computing device to another computing device, data representative of incorrect performance of the predetermined physical exercise when at least one criterion of the predetermined set of criteria has not been fulfilled; or
modifying, by the at least one computing device, the criteria by disabling one or more criteria thereof, or by changing values of one or more criteria thereof.

11. The method of any one of the preceding claims, wherein the one or more instructions or signals are indicative of at least one of:
which criterion have not been fulfilled; and
guidance on how to move the one or more body members or the pelvic floor muscle to fulfill the criterion that have not been fulfilled.

12. The method of any one of the preceding claims, wherein at least one of the first measurements or further measurements are taken by the at least one optical sensor, and the method further comprises processing, by the at least one computing device, the at least one of the first measurements or further measurements taken at least while the person performs the predetermined physical exercise.

13. The method of any one of the preceding claims, wherein the pelvic sensor further measures motion of a pelvic floor of the person, wherein the determination of whether the person has performed the predetermined physical exercise further comprises evaluating the processed second measurements to determine whether motion of the pelvic floor fulfills at least one further criterion of the criteria.

14. The method of any one of the preceding claims, wherein:
the predetermined physical exercise further comprises lack of motion of at least one body member of the person;
the first measurements further measure motion of the at least one body member; and
determining whether the person has performed the predetermined physical exercise further comprises evaluating the processed first measurements to determine whether the motion of the at least one body member fulfills at least one criterion of the criteria associated with lack of motion.

15. A motion tracking system comprising:
at least one motion tracking device or at least one optical sensor;
a pelvic sensor; and
a computing device comprising a processor operative to:
process first measurements taken at least while a person performs a predetermined physical exercise,
wherein the first measurements are taken by at least one motion tracking device, at least one optical sensor, or both;
process second measurements taken at least while the person performs the predetermined physical exercise, wherein the second measurements are taken by a pelvic sensor; and
determine whether the person has performed the predetermined physical exercise based on the first measurements and the second measurements according to criteria associated with the predetermined physical exercise.
